# EUROPEAN PATENT SPECIFICATION

(11) **EP 2 737 798 B1**
(45) Date of publication and mention of the grant of the patent: **14.08.2019**
(21) Application number: 13192329.4
(22) Date of filing: 11.11.2013
(51) Int. Cl.: A01N 63/00, A01N 25/08, A01P 15/00, B64D 1/18, A01M 9/00, A01K 67/033

(54) **METHOD, DEVICE AND MIXTURE FOR AERIAL APPLICATION OF TRICHOGRAMMA PARASITOIDS IN PLANT PROTECTION**
VERFAHREN, GERÄTE-UND GEMISCH ZUR LUFTANWENDUNG VON TRICHOGRAMMA-SCHLUPFWESPEN IM PFLANZENSCHUTZ
PROCÉDÉ, DISPOSITIF ET MÉLANGE POUR L'ÉPANDAGE AÉRIEN DES PARASITOÏDES TRICHOGRAMMES DANS LA PROTECTION DES VÉGÉTAU

(30) Priority: 28.11.2012 CZ 20120851
(43) Date of publication of application: 04.06.2014
(73) Proprietor: Biocont Laboratory, spol. s r.o., 664 42 Modrice (CZ)
(72) Inventor: Hluchý, Milan, 66432 Vranov (CZ)
(74) Representative: Musil, Dobroslav

(56) References cited:
- US-A- 5 794 847
- Anonymous: "Trichogramma release methods", , 14 February 2006 (2006-02-14), XP055101384, Retrieved from the Internet: URL:https://web.archive.org/web/2006021408 5742/http://www.bioresources.com.au/Pretio sum/PretiosumMethods.htm [retrieved on 2014-02-11] & Anonymous: "Pretiosum - General Information", , 14 February 2006 (2006-02-14), XP055101500, Retrieved from the Internet: URL:https://web.archive.org/web/2006021408 5712/http://www.bioresources.com.au/Pretio sum/PretiosumPrices.htm [retrieved on 2014-02-11]
- E. J. Lockett: "Sticky Seed Traps-A useful Tool for Monitoring Seed Distribution During Aerial Sowing", Tasforests, 30 September 1994 (1994-09-30), pages 23-31, XP055129493, Retrieved from the Internet: URL:https://cdn.forestrytasmania.com.au/as sets/0000/0451/article_3.pdf [retrieved on 2014-07-17]
- Anonymous: "Trichogramma Release strategies", , 14 February 2006 (2006-02-14), XP055245463, Retrieved from the Internet: URL:https://web.archive.org/web/2006021408 5812/http://www.bioresources.com.au/Pretio sum/PretiosumStrategies.htm [retrieved on 2016-01-27]

## Description

### Technical field

The invention relates to a method of aerial application of parasitoids of the genus Trichogramma in plant protection, in which a defined amount of Trichogramma parasitoids in defined developmental stages is released from flying means on a defined plant surface area or a unit of plant surface, while released Trichogramma parasitoids is mixed with semolina into loose inert substrate of natural origin and dispensing is performed by dispenser with gravity reservoir of loose inert substrate to dispensing mechanism with carrier mounted on output shaft of an engine and with rotating perforated dosing disc with adjustable revolutions.

The invention further relates to a device for aerial application of Trichogramma parasitoids in plant protection, which comprises a gravity reservoir of loose mixture of Trichogramma parasitoids pupae in defined developmental stages and semolina and further comprises a dispensing mechanism of the loose mixture with carrier mounted on output shaft of an engine and with rotating perforated dosing disc with adjustable revolutions.

The invention also relates to a loose mixture for aerial application of Trichogramma parasitoids in plant protection, which is composed of the pupae of the Trichogramma parasitoid in defined developmental stages and of a loose inert substrate of natural origin.

### Background art

Trichogramma egg parasitoids are specialized in parasitization of eggs of hosting species of insects, particularly butterflies. Due to this ability they have been used in the sphere of biological plant protection for several decades, i.e. they have been acting as biological control agents against pests, namely against caterpillars of butterflies. Nowadays, Trichogramma parasitoids are most widely applied in the sphere of maize protection from the European corn borer. Trichogramma parasitoids are used to control this pest in such a manner that a defined amount of the pupae of the parasitoid per unit of the maize field is distributed over the maize field during the initial phase of laying eggs by a particular pest, i.e. in this case for example by females of the European corn borer. After females of the parasitoid hatch from the pupae of the parasitoid, they actively, by smelling within a limited action area (tens of centimetres up to individual metres), search for clusters of eggs laid by the pest. The females of the parasitoid lay their own eggs into the eggs of the pest, whereupon inside this parasitized egg of the pest (the host) a parasitoid of the genus Trichogramma completely develops in a relatively short period of time - in approximately 10 days, whereby, simultaneously, the embryo of the pest is destroyed, i.e. the embryo of the caterpillar of the European corn borer, by which means the occurrence of the European corn borer in the maize field treated is eliminated, and, consequently, the maize field is in this manner protected from this pest and the plant surface area thus protected is not damaged.

As Trichogramma parasitoids due to the extreme miniaturization of their body size hatch only with minimum energy reserves (fats, sugar) and move mainly by walking short distances (tens of centimetres up to individual metres), it is necessary to distribute a sufficient amount of Trichogramma parasitoids per unit of the plant surface area treated in order to ensure that each plant of the field treated is subjected to the acting of Trichogramma parasitoids, which have a very limited action radius, since Trichogramma parasitoids have to have enough energy for their existence to be able to search for the clusters of the pest's eggs (hosting eggs) on the plants and to be able to parasitize them.

Nowadays, there are several methods of the application of Trichogramma parasitoids. The first known method of the application of Trichogramma parasitoids is dropping or throwing the pupae of the parasitoids into the axil of the maize leaves by hand, whereby the pupae of the parasitoid in the form of "powder" have a particle size of approximately 0.7 mm. A second known method of the application of Trichogramma parasitoids is the placing of a defined amount of Trichogramma parasitoids on paper cards or into capsules of various constructions, which are subsequently distributed again by hand in the maize field in accordance with the requirements per unit of the area of the plot protected.

However, these methods have a number of disadvantages which can be roughly divided into two categories: organizational and technical problems and biological disadvantages.

The organizational and technical problems are connected with financial and organizational demands of plant protection, as it is highly labour intensive, when 1.5 man hour/ha is needed for the protection of the area. Owing to the fact that the biological plant protection by means of Trichogramma parasitoids requires relatively precise timing of the application of the parasitoids, and, what is more, due to the fact that the suitable time for the application is approximately 2 - 3 days from the point of view of the time point of the beginning of laying the eggs by the particular pest, it is practically impossible to treat large plant surface areas, such as maize fields, sometimes covering areas of up to thousands of hectares. In the case of such vast plant surface areas protected, using Trichogramma parasitoids for biological protection would require employing an enormous quantity of labourers in an extremely short period of time, which brings not only enormous workforce costs, but also involves costs to cover the workforce activization, accommodation and other living conditions.

The biological disadvantages include the occurrence of unprotected clusters of a relatively large amount (approximately 1,000 - 1,500 p. in one place) of the pupae of the parasitoids of Trichogramma parasitoids in one place of the field protected using the existing decscribed methods of application of Trichogramma parasitoids on the plant surface area, since Trichogramma parasitoids are highly attractive for predaceous insects (lady birds, predaceous bugs), which causes considerable losses in the order of as many as tens of percent during the time period from the application of Trichogramma parasitoids to the hatching of Trichogramma parasitoids from the pupae, which both increases the costs of biological plant protection by means of Trichogramma parasitoids, and decreases the efficiency of biological plant protection by means of Trichogramma parasitoids. Although this biological drawback is reduced by the encapsulation of the pupae of Trichogramma parasitoids into capsules, this does not solve the existence of another major shortcoming, namely the demanding and slow distribution of Trichogramma parasitoids on the plant surface, e.g. in maize fields.

There are also known methods, devices and means for aerial application of a parasitoid of the genus Trichogramma, for example according to US patents No 3 872 012, 3 968 933, 3 994 437, 4 089 441, 4 260 108, 4 475 819, 4 487 365, 4 966 329, 5 148 989, 5 190 225, 5 213 271, 5 288 028, 5 794 847, 4 537 333 and others.

In principle, they describe different solutions to the problem of releasing biologically active insects in required growth stages, for example Trichogramma parasitoids, and the maximum possible uniform distribution on the area treated. For this purpose, a variety of applicators is used, which comprise a dispenser connected to a "spraying" device, into which during the flight of the aircraft the surrounding air gets spontaneously, which evokes a stir-up of the individuals of biologically active insects in the air in the spraying device and by the subsequent releasing of this mixture of the air and the biological material from the spraying device the biological material is distributed on the area treated. These solutions are demanding in terms of design and construction, as well as operation.

According to one of the above-mentioned patents, the biologically active insects are treated with a layer of adhesive material so that the sprayed biological material is better captured on the plants, by which means the method is supposed to be more efficient. However, this makes the whole method more expensive and, in addition, the practical effect of the adhesive material on the individuals of the sprayed insects after hatching from the pupae is disputable.

This document also describes a method of evaluation of the quality of distribution of the applied biologically active insects by means of a long stripe of capturing material divided by marks into sections one meter long located on plywood units and cardboard, which are carried to the place of the insect application in plastic vessels and are distributed together with leaves of walnuts into the monitored area of the insect application. After that an aircraft with a set dispenser of biologically active insects flies over the given area and by means of a hand lens (7 times enlargement) the number of captured individuals of the sprayed insects is evaluated. Simultaneously, the capturing material and the walnut leaves are taken to a laboratory for 7-day incubation. Afterwards another detailed analysis of the presence of the eggs of the applied insects - the parasites - is carried out, and according to the number of eggs with holes the percentage of successful hatching of an individual of the applied insects per unit area of the capturing material is determined, which is essential for the overall assessment of the efficiency of the application of biologically active insects in the field treated. This method is extremely complicated, time-consuming and requires special equipment and is not able to provide relevant data about the quality of the application immediately and on the spot of the application.

Web pages www.biorecources.com.au discloses a process for hand spreading of TG (Trichogramma) capsules, while each capsule contains 1000 pupae. Per 1 ha of surface treated is to be delivered from 60 to 120 TG capsules. Such application is possible at low crops, respectively in vegetables, when a worker manually places each capsule with TG in a certain geometrical pattern. The disadvantage is a considerable time-consuming and labor intensive. US 5 794 847 A teaches, that such carriers essentially represent large concentration of TG eggs on one place which attracts insects, which reduces the final amount of TG to be able to fulfill its protective role. Since the shape and size of the trees is random, it is substantially impossible to achieve the necessary distribution of the TG, so that some areas will be oversaturated by occurrence of TG and in some areas will be only a few TG individuals.

From the web pages www.biorecources.com.au is also known to discharge loose mixture of TG pupae and semolina at concentration 1:10 from aircraft, where 1 I of this mixture should suffice for 50 ha of treated area. The bottle with the mixture is screwed into the dispensing line in the aircraft cabin and dispensing of the mixture is manually operated from the aircraft cabin. Manual control of TG dispensing is completely inappropriate, because there is an uneven distribution of TG pupae on treated area, so local places where the TG pupae is in too huge concentration and places where the TG pupae is in too low concentration are made. Such a basically manually operated dispensing of TG by aircraft is highly inefficient and unreliable.

From US5794847A is known a method for aircraft application of TG when a mixture of TG pupae and binder is transported into the sprayer and therein into a sufficiently fast flow of air, which ensures the creation of the spray effect. This technique is applied to either the aircraft or ground vehicle with enough powerful generator of air flow (fan). A necessary condition for the solution according to US5794847A is that the TG is before the dispersing mixed with a binder, e.g. glue (mucilage), so that the apparatus according US5794847A is spraying "sticky" TG pupae coated with glue or other non-lethal binder in order to attach the TG on the desired tree or other protected plant. US5794847A describes how a hopper measures out a defined amount of TG into the collecting vessel, from which via the nozzle by gravity is supplied to the J-tube and further into the chamber for mixing with the glue. From US5794847A is thus known aerial application of TG pupae at a certain stage of development while the TG pupae themselves are placed in the container from which they are conveyed into an artificial air flow (fan or the ramjet through rapid movement of the aircraft) into which is fed non-lethal binder, e.g. mucilage that covers TG pupae to ensure that pupae "sticks" to the point at which they fall from aircraft. Non-lethal binder, however, has a major drawback of the method consisting in influencing of TG individual hatching from TG pupae coated by binder etc. Another disadvantage is the high complexity of the device for performing the method, which requires simultaneous precisely controlled dosage of both TG pupae and binder to artificially generated air stream.

From web pages www.biorecources.com.au isalso known spreading of a certain amount of TG pupae per unit of area of the protected crop, wherein the numbers of pupae oscillate between 60 thousand and 120 thousand per hectare according to the kind of protected crop.

The aim of the invention is to eliminate or at least minimize the disadvantages of the background art in the sphere of aerial application of Trichogramma parasitoids, and, above all, to enable fast, functional, efficient and checkable distribution of Trichogramma parasitoids in on the plant surface area protected in the required amount and at the required time, all this being performed under acceptable economic conditions.

### Principle of the invention

The goal of the invention is achieved by a method of the application of Trichogramma parasitoids in plant protection, whose principle consists in that from 100,000 to 250,000 individuals of the Trichogramma parasitoid on a treated area of 1 ha of plant surface is released, while the loose mixture is released in incremental doses from dents on the circumference of the dosing disc on the output shaft of the engine directly to the air and falls down onto the treated area of the crops..

The principle of the device for the application of Trichogramma parasitoids in plant protection consists in that the the dosing disc is on its circumference equipped with dents for measuring the released doses of the loose mixture, while the loose mixture is freely discharged from the dents to the air by rotation of the dosing disc for free falling down onto the treated area of the crops.

The principal of the mixture for application of Trichogramma parasitoids in plant protection consists in that it has concentration of 18 % Trichogramma and 82 % semolina.

The advantage of this invention is a possibility to treat even vast areas of vegetation quickly, reliably and with a defined presicion, whereby it is possible to achieve high efficiency of biological protection of plants at relatively low costs.

### Description of drawings

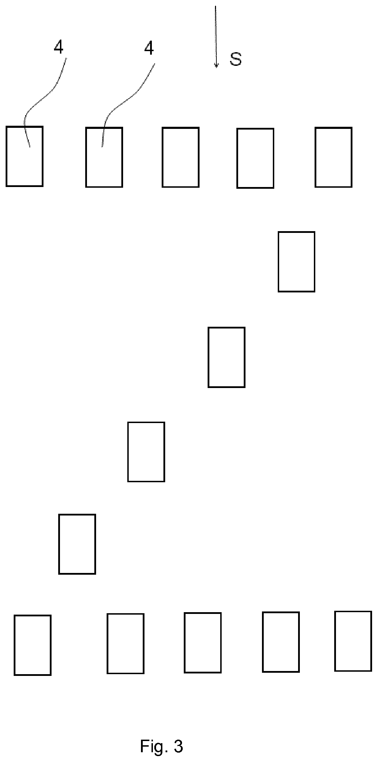

The invention is schematically represented in the drawing, where Fig. 1 shows the structure of the loose mixture, Fig. 2a is a side view of a schematic arrangement of the dispensing mechanism of the loose mixture, Fig. 2b is a top view of a schematic arrangement of the dispensing mechanism of the loose mixture without a gravity reservoir of the loose mixture and Fig. 3 shows the distribution of the control capturing pads for the evaluation of the application.

### Specific description

The invention will be described on an example of the application of Trichogramma parasitoides in a loose mixture with a loose inert substrate of natural origin, where the application is carried out by means of a device for the application of the loose mixture for plant protection, whereby the device for the application of the loose mixture is attached to a flying means, e.g. an airplane, and the quality and/or homogeneity of the application is evaluated by a method according to the present invention.

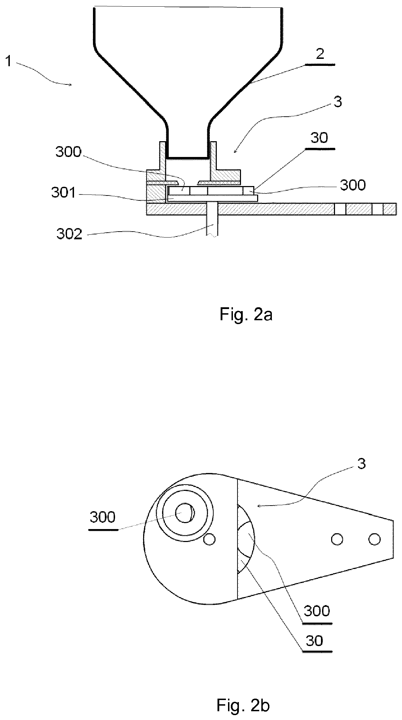

The device for the application of Trichogramma parasitoids in this loose mixture in plant protection (see Fig. 2) comprises a dispenser **1** of loose materials with a gravity reservoir **2** of the loose mixture.

The loose mixture includes a loose inert substrate of natural origin and the pupae of the parasitoid Trichogramma in precisely defined developmental stages, which determine the dynamics of hatching of the parasitoid in time. The loose inert substrate of natural origin includes, for example, barley corn or a mixture of barley corn and other grain products or some other material of natural origin, whereby the reason why the loose inert substrate of natural origin according to this invention is used is the fact that it has no negative influence on the pupae of the parasitoid Trichogramma, nor has it any negative impact on the environment in the area of the aerial application of the loose mixture of the pupae of the parasitoid Trichogramma and the loose inert substrate of natural origin. Another reason for employing the loose inert substrate of natural origin according to the present invention is the fact that it ensures the required physical parameters of the resulting loose mixture, especially its looseness etc. In order to achieve and maintain the physical properties of the resulting loose mixture it is advantageous to use a loose inert substrate having equal size grains or grains whose size is similar to that of the pupae of the parasitoid Trichogramma.

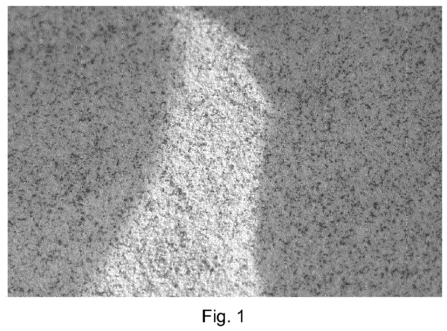

When preparing the loose mixture according to the invention a required rate of individual components of the loose mixture is set, i.e. the rate of the pupae of the parasitoid Trichogramma and the loose inert substrate of natural origin according to the required amount of the individuals of the parasitoid Trichogramma on a unit of area of the future protected plot of vegetation, for example 120,000 - 150,000 individuals/ha, which leads to the formation of a mixture of the pupae of the parasitoid and the loose inert substrate having a required concentration of the pupae of the parasitoid in a unit of capacity of the resulting loose mixture, which is shown in Fig. 1.

The resulting loose mixture of the pupae of the parasitoid Trichogramma and of the loose inert substrate of natural origin is inserted into the reservoir **2** of the loose mixture of the dispenser **1**. The dispenser **1** is adjustable in relation to the amount of the loose mixture given out per unit of time and is located on the flying means, such as an airplane. According to the concentration of the parasitoid of the genus Trichogramma in the resulting loose mixture and according to the required quality and/or uniformity of the application in accordance with the current meteorological conditions, such as the power and direction of the wind, humidity, etc., the required speed is determined, the altitude and direction of the flight of the flying means with an installed dispenser **1**, which is equipped with means of fastening to a suitable part of the aerial means, such as an airplane, a helicopter, remotely controlled airplane, an automatic aerial means, etc., for example to a landing gear or a fuselage , or under a wing or wings, to a tail, or to another suitable part of the flying means etc. One flying means may comprise one dispenser **1** or more dispensers **1**, e.g. two or three, etc., attached to it.

The drawing shows a dispenser **1** of the resulting loose mixture of the pupae of the parasitoid Trichogramma and the loose inert substrate of natural origin, which is provided with a gravity dispenser **2** of the loose mixture of the pupae of the parasitoid Trichogramma and the loose inert substrate of natural origin. Assigned to the lower end of the gravity reservoir **2** is a dispensing mechanism **3** of the loose mixture with a rotating dosing disc **30** for measuring individual incremental doses of the loose mixture of the pupae of the parasitoid Trichogramma and of the loose inert substrate of natural origin. The dosing disc **30** is loosely slipped over a carrier **301**, which is mounted on an output shaft **302** of an unillustrated engine. On the circumference of the dosing disc **30** there are five dents **300** for measuring the delivered dose of the loose mixture, and so during the rotation of the dosing disc **30** the dents **300** are gradually filled with material from the gravity reservoir **2** of the loose mixture. During the further rotation of the dosing disc **30** the measured loose mixture is freely discharged from the dents **300** to the air and falls down onto the treated area of the crops. Apart from the regulation of the speed and altitude of the flight, the speed and amount of the released loose mixture can be regulated by the speed of releases and the amount of the released mixture, which can be performed in the illustrated embodiment of the dispenser **1** by setting the revolutions of the rotating dosing disc **30** and/or by changing the size of the dents **300** of the dosing disc **30**, for example by replacing one dosing disc **30** with a certain number of dents **300** of a certain size with another perforated disc **30** with a different number of and/or a different size of the dents **300**.

The homogeneity and quality of the application of the required amount of the pupae of Trichogramma parasitoids per unit area of the plant surface treated can be verified by a method according to the invention, in which on a free area, such as the area of an airport, etc., or directly on the protected plant surface, evenly in a defined mutual distance, e.g. 1m, perpendicularly to the direction of the flight of the flying means with a determined number of dispensers **1,** capturing pads **4** of the loose mixture are distributed, e.g. it is possible to use a5 size plastic plates provided with a sticky coating on their upper surfaces, whereby the capturing pads **4** can be preferably any colour except black, since the evaluated pupae of Trichogramma parasitoids are dark or black, and the colour of the loose inert substrate of natural origin is usually light (including the range of white to yellow and light brown).

In order to increase the information value of the evaluation of the homogeneity and quality of the application the capturing pads **4** are distributed in a geometrically suitable arrangement in relation to the direction of the flight **S** of the flying means, e.g. in a matrix arrangement, or in the form of a letter of the alphabet, especially the form of the letter "Z" is advantageous, etc. These capturing pads **4** are evaluated after the flight of the flying means by the naked eye or with the aid of a magnifying glass regarding the number of the pupae of Trichogramma parasitoids caught on individual capturing pads **4**. In this manner it is possible to verify the quality and homogeneity of just performed application immediately on the spot after the application of the pupae of Trichogramma parasitoids, i.e. to verify the amount of the pupae of Trichogramma parasitoids per unit area and also check the homogeneity of the performed application and, if needed, e.g. in case the required homogeneity and amount have not been achieved due to an abrupt climatic change (a sudden gust of wind) it is possible to carry out without delay a repeated application with the same or modified parameters of the application, such as the speed and altitude of the flight, the amount of the released loose mixture per unit of time etc. It is apparent that the flying means flies over the treated area there and back in gradually shifting stripes, by which means the application is performed on the entire area of the plant surface treated.

By setting the concentration of the loose mixture appropriately, as well as by setting the amount of the loose mixture dosed by the dispenser **1** per unit of time, the speed and altitude of the flight of the particular type of aircraft used, etc., it is possible to achieve the application of a verified and guaranteed number of the pupae of Trichogramma parasitoids per unit area of the plant surface treated , e.g. 100.000 - 250.000 of individuals/ha. In order to achieve a more precise navigation of the flight of the flying means, especially in order to prevent the occurrence of untreated spots due to not observing the maximum allowed distance between individual flights of the flying means over the plant surface area treated, it is advantageous to use a suitable positioning system, for example the system GPS, GLONASS, GALILEO etc., which brings another benefit, namely the precise control of the flight with the possibility of its automation, and also the possibility of recording the flight path, including individual transits in individual stripes over the plant surface treated, which, for example, enables feedback control of the flight path, etc.

It follows from the applicant's experience that the optimum application of the pupaes of Trichogramma parasitoids constitutes 1 pupa of the Trichogramma parasitoid per approximately every 7 dm² of the area of the plant surface treated. For example, when seeding maize with the spacing 60 x 10 cm the average distance of the hatched individual of a Trichogramma parasitoid to the most distant plant of maize, which each individual of a Trichogramma parasitoid is to "search",is in the order of decimetres, which is an optimum state, leading to the 75 - 95 % efficiency of protection according to this invention. This density of distribution of the pupae of Trichogramma parasitoids considerably reduces the risk of the individuals of Trichogramma parasitoids being found and destroyed by predators, such as ladybird beetles, predaceous bugs, ants, etc.

As an experiment, the applicant achieved the treatment of a field having an area of approximately 4.3 ha/minute, the price of the application being approximately 140 crowns/ha, the real application capacity being approximately 1.000 ha/ 8 hrs., using one agricultural aicraft. These values are experimental values, achieved with the illustrated embodiment of the dispenser in Fig. 2, with a particular concentration of the loose mixture (18 % trichogramma and 82 % semolina) and with the aid of a particular type of aircraft (Z 37A Čmelák). It is obvious that by the appropriate setting of these parameters, it is possible to influence the areal application capacity.

For example, to meet the requirement of applying 120,000 - 150,000 individuals of the Trichogramma parasitoid on a treated area of 1 ha, the selected type of the loose mixture will have the concentration of 18 % Trichogramma and 82 % semolina, and at the selected speed of the flying means or at the speed which is known beforehand according to the type of the flying means and depending on the selected altitude of the flight of 4 - 6 m over the ground, the set amount of the released loose mixture per 1 minute will be 100 ml, and so a dosing disc **30** will be used, which is 8 mm thick and whose diameter is 48 mm and which has 5 dents **300** with a diameter of 20 mm. Under these conditions the application of the required amount of individuals of the Trichogramma parasitoid on the treated area is achieved. It is apparent that the varying factors include especially the concentration of the loose mixture, the speed of the flight of the flying means and the speed and amount of the released loose mixture. If needed, it is possible to use a flying means with two or more dispensers **1.**

## Claims

1. A method of aerial application of parasitoids of the genus Trichogramma in plant protection, in which a defined amount of Trichogramma parasitoids in defined developmental stages is released from flying means on a defined plant surface area or a unit of plant surface, while released Trichogramma parasitoids is mixed with semolina into loose inert substrate of natural origin and dispensing is performed by dispenser (1) with gravity reservoir (2) of loose inert substrate to dispensing mechanism (3) with carrier (301) mounted on output shaft (302) of an engine and with rotating perforated dosing disc (30) with adjustable revolutions, **characterized in that** from 100,000 to 250,000 individuals of the Trichogramma parasitoid on a treated area of 1 ha of plant surface is released, while the loose mixture is released in incremental doses from dents (300) on the circumference of the dosing disc (30) on the output shaft (302) of the engine directly to the air and falls down onto the treated area of the crops.

2. A method according to Claim 1, **characterized in that** from 120,000 to 150,000 individuals on a treated area of 1 ha is released.

3. A method according to Claim 2, **characterized in that** 120,000 to 150,000 individuals on a treated area of 1 ha is released during flight in altitude of 4 - 6 m over the ground, while the individuals is released as 100 ml of loose mixture of concentration of 18 % Trichogramma and 82 % semolina per 1minute of flight and the loose mixture is released by dosing disc (30) of thick 8 mm, diameter 48 mm, which has with 5 dents (300) with diameter of 20 mm situated on the circumference of the dosing disc (30).

4. A method according to claims 1 to 3, in which before the application of the loose mixture consisting of the pupae of the parasitoid Trichogramma and of a semolina, a system of small capturing pads (4) of the loose mixture with sticky coating on their upper surfaces is distributed on a defined area of the plant surface, whereupon the application of Trichogramma parasitoids with the aid of the flying means is carried out and subsequently the number of the pupae of Trichogramma parasitoids caught on individual capturing pads (4) is optically evaluated, **characterized in that** capturing pads (4) are formed by A5 size plastic plates and are arranged in the form of a letter "Z".

5. A device for aerial application of Trichogramma parasitoids in plant protection according to claim 1, which comprises a gravity reservoir (2) of loose mixture of Trichogramma parasitoids pupae in defined developmental stages and semolina and further comprises a dispensing mechanism (3) of the loose mixture with carrier (301) mounted on output shaft (302) of an engine and with rotating perforated dosing disc (30) with adjustable revolutions **characterized in that** the dosing disc (30) is on its circumference equipped with dents (300) for measuring the released doses of the loose mixture, while the loose mixture is freely discharged from the dents (300) to the air by rotation of the dosing disc (30) for free falling down onto the treated area of the crops.

6. A device according to Claim 5, **characterized in that** the dosing disc (30) is replaceable with dosing disc (30) different number of dents (300) and/or a different size of the dents (300).

7. A loose mixture for the aerial application of Trichogramma parasitoids in plant protection according to claims 1 to 6, which is composed of the pupae of the Trichogramma parasitoid in defined developmental stages and of a loose inert substrate of natural origin, **characterized in that** has concentration of 18 % Trichogramma and 82 % semolina.

## Patentansprüche

1. Verfahren zur Flugapplikation von Parasitoiden der Gattung Trichogramma im Pflanzenschutz, bei dem die definierte Menge von Einzelwesen von Parasitoiden der Gattung Trichogramma in definierten Entwicklungsphasen aus dem fliegenden Mittel auf eine definierte Bewuchsfläche oder auf eine Einheit der Bewuchsfläche ausgelassen wird, wobei der auszulassende Parasitoid der Gattung Trichogramma mit einem Getreidegrieß zu einem lockeren Inertsubstrat einer Naturherkunft gemischt wird und die Dosierung durch einen Dosierer (1) mit einem Schwerkraftbehälter (2) des lockeren Inertsubstrates in die Dosiereinrichtung (3) mit einem Mitnehmer (301), der auf der Ausgangswelle (302) der Motors befestigt ist, und mit einer perforierten rotierenden Dosierscheibe (30) mit einstellbaren Drehzahlen erfolgt, **dadurch gekennzeichnet, dass** 100.000 - 250.000 Einzelwesen der Gattung Trichogramma auf die zu behandelnde Fläche von 1 ha der Bewuchsfläche ausgelassen werden, wobei die lockere Mischung in den inkrementalen Dosierungen aus den Einschnitten (300) auf dem Umfang der Dosierscheibe (30) auf der Ausgangswelle (302) der Motors direkt in die Luft ausgelassen wird und auf die zu behandelnde Pflanzenfläche nach unten fällt.

2. Verfahren nach dem Anspruch 1, **dadurch gekennzeichnet, dass** 120.000 bis 150.000 Einzelwesen auf die zu behandelnde Fläche von 1 ha ausgelassen werden.

3. Verfahren nach dem Anspruch 2, **dadurch gekennzeichnet, dass** 120.000 bis 150.000 Einzelwesen auf die zu behandelnde Fläche von 1 ha beim Flug in der Höhe von 4 - 6 m über Terrain ausgelassen werden, wobei die Einzelwesen als 100 ml der lockeren Mischung mit einer Konzentration von 18 % Trichogramma und 82 % Grieß pro 1 Flugminute ausgelassen werden und die lockere Mischung durch eine Dosierscheibe (30) mit einer Stärke von 8 mm, Durchmesser von 48 mm und mit 5 Einschnitten (300) mit einem Durchmesser von 20 mm ausgelassen wird, die auf dem Umfang der Dosierscheibe (30) situiert sind.

4. Verfahren nach Ansprüchen 1 bis 3, bei dem vor der Applikation der Puppen des Parasitoids Trichogramma und Getreidegrieß enthaltenden lockeren Mischung auf die definierte Fläche des zu behandelnden Bewuchses der lockeren Mischung ein System der Auffangflächen (4) der lockeren Mischung mit einer klebrigen Beschichtung auf ihrer oberen Fläche hinbreitet wird, wonach die Applikation von Parasitoiden der Gattung Trichogramma mit Hilfe des Flugmittels durchgeführt wird und anschließend die Anzahl von Puppen der Parasitoiden der Gattung Trichogramma ausgewertet wird, die auf einzelnen Auffangflächen (4) aufgenommen werden, **dadurch gekennzeichnet, dass** die Auffangflächen (4) durch Kunststoffplatten des Formats A5 gebildet werden und in der Form des Buchstabens "Z" angeordnet sind.

5. Einrichtung zur Flugapplikation von Parasitoiden der Gattung Trichogramma im Pflanzenschutz laut Anspruch 1, die einen Schwerkraftbehälter (2) der lockeren Mischung der Puppen des Parasitoids Trichogramma in definierten Entwicklungsphasen und des Getreidegrießes aufweist und die weiter einen Dosierer (3) der lockeren Mischung mit einem Mitnehmer (301), der auf der Ausgangswelle (302) des Motors gelagert ist, und mit einer rotierenden perforierten Dosierscheibe (30) mit einstellbaren Drehzahlen aufweist, **dadurch gekennzeichnet, dass** die Dosierscheibe (30) auf ihrem Umfang die Einschnitte (300) zur Abmessung der ausgelassenen Dosierung der lockeren Mischung aufweist, wobei die lockere Mischung aus den Einschnitten (300) in die Luft durch Drehung der Dosierscheibe (30) zum freien Fallen auf die zu behandelnde Pflanzenfläche frei ausgelassen wird.

6. Einrichtung nach dem Anspruch 5, **dadurch gekennzeichnet, dass** die Dosierscheibe (30) gegen eine andere Dosierscheibe (30) mit einer anderen Zahl von Einschnitten (300) und/oder mit einer anderen Größe von Einschnitten (300) getauscht werden kann.

7. Lockere Mischung zur Flugapplikation von Parasitoiden der Gattung Trichogramma im Pflanzenschutz nach den Ansprüchen 1 bis 6, die durch Puppen des Parasitoids Trichogramma in definierten Entwicklungsphasen und lockeres Inertsubstrat einer Naturherkunft gebildet wird, **dadurch gekennzeichnet, dass** diese die Konzentration von 18 % Trichogramma und 82 % Getreidegrieß aufweist.

## Revendications

1. Procédé de l'application aérienne des parasitoïdes du genre Trichogramma pour la protection des plantes dans laquelle le nombre défini des parasitoïdes du genre Trichogramma dans les stades du développement définis est déchargé d'un appareil volant sur la surface définie des plantes ou sur l'unité de la surface des plantes, tandis que le parasitoïde déchargé du genre Trichogramma est mélangé avec la semoule des céréales pour faire un substrat inerte d'origine naturelle et le dosage est effectué par un distributeur (1) avec un réservoir de gravitation (2) d'un substrat inerte en vrac dans le mécanisme de dosage (3) avec un support (301) monté sur l'arbre de sortie (302) du moteur et avec un disque de distribution perforé rotatif (30) à vitesse réglable **caractérisé en ce que** de 100 000 à 250 000 individus de Trichogramma sont déchargés dans la surface traitée d'un hectare des plantes, tandis que le mélange en vrac est déchargé par doses incrémentielles des encoches (300) situées à la périphérie du disque de distribution (30) sur l'arbre de sortie (302) du moteur directement dans l'atmosphère et il tombe sur la surface traitée des plantes.

2. Procédé selon la revendication 1, **caractérisé en ce qu'**on décharge de 120 000 à 150 000 individus par hectare de la zone traitée.

3. Procédé selon la revendication 2, **caractérisé en ce qu'**on décharge de 120 000 à 150 000 individus par zone traitée de 1 hectare pendant le vol à une hauteur de 4 à 6 m au-dessus du sol, tandis que les individus sont déchargés sous forme de 100 ml d'un mélange en vrac avec la concentration de 18 % de trichogrammes et de 82 % d'une semoule par minute de vol et le mélange en vrac est évacué par un disque de distribution (30) avec l'épaisseur de 8 mm, avec le diamètre de 48 mm et avec 5 encoches (300) du diamètre de 20 mm situées sur le circuit du disque de distribution (30).

4. Procédé selon les revendications 1 à 3, pendant lequel, avant l'application de la composition en vrac comprenante les pupes du parasitoïdes Trichogramme et la semoule des céréales, l'ensemble des petites surfaces de capture (4) d'un mélange en vrac avec un revêtement collant sur sa surface supérieure est répartis sur la zone définie des plantes traitées par le mélange en vrac, après quoi l'application des parasitoïdes du genre Trichogramma est effectuée à l'aide d'un appareil volant et le nombre des parasitoïdes du genre Trichogramma capturés sur les différentes surfaces de capture (4) est ensuite évalué visuellement, **caractérisé en ce que** les petites surfaces de capture (4) sont formées de feuilles de plastique A5 et elles sont disposées sous la forme de la lettre "Z".

5. Dispositif pour l'application aérienne des parasitoïdes du genre Trichogramma dans la protection des plantes selon la revendication 1, qui comprend un réservoir de gravitation (2) d'un mélange en vrac des pupes parasitoïdes de Trichogramme à des stades du développement définis et des semoules des céréales et comprenant en outre un distributeur (3) du mélange en vrac avec le support (301) monté sur l'arbre de sortie du moteur (302) et un disque de distribution perforé rotatif (30) à vitesse réglable, **caractérisé en ce que** le disque de distribution (30) à sa périphérie est pourvu des encoches (300) pour mesurer la dose distribuée du mélange en vrac, tandis que le mélange en vrac est évacué librement des encoches (300) dans l'air en faisant tourner le disque de distribution (30) pour la chute libre sur la zone traitée des plantes.

6. Dispositif selon la revendication 5, **caractérisé en ce que** le disque de distribution (30) peut être remplacé par un autre disque de distribution (30) avec un nombre différent des encoches (300) et/ou des encoches différentes (300).

7. Mélange en vrac pour l'application aérienne des parasitoïdes du genre Trichogramma en protection des plantes selon les revendications 1 à 6, qui comprend les pupes du parasitoïde Trichogramma dans les phases du développement définies et un substrat inerte en vrac d'origine naturelle, **caractérisée en ce qu'**il a une concentration de 18 % de Trichogramma et de 82 % de la semoule des céréales.
